# EUROPEAN PATENT APPLICATION

(11) **EP 1 872 708 A1**
(43) Date of publication of application: **02.01.2008**
(21) Application number: 07000059.1
(22) Date of filing: 03.01.2007
(51) Int. Cl.: A61B 1/018

(54) **Endoscope treatment system, treatment instrument, and adapters**

(30) Priority: 23.06.2006 US 473819
(71) Applicant: OLYMPUS MEDICAL SYSTEMS CORP., Shibuya-ku Tokyo 151-0072 (JP)
(72) Inventor: Murakami, Kazushi Olympus Medical Systems Corp., Shibuya-ku Tokyo 151-0072 (JP); Onuki, Yoshio Olympus Medical Systems Corp., Shibuya-ku Tokyo 151-0072 (JP); Komiya, Takaaki Olympus Medical Systems Corp., Shibuya-ku Tokyo 151-0072 (JP); Ichikawa, Hiroaki Olympus Medical Systems Corp., Shibuya-ku Tokyo 151-0072 (JP); Kura, Yasuhito Olympus Medical Systems Corp., Shibuya-ku Tokyo 151-0072 (JP); Nishiie, Takehiro Olympus Medical Systems Corp., Shibuya-ku Tokyo 151-0072 (JP)
(74) Representative: von Hellfeld, Axel

(57) **Abstract**

An endoscope treatment system comprises plural types of treatment instruments, plural types of adapters, and an advancing and retracting device. The treatment instmments include treatment instrument inserting sections which can be inserted into a treatment instrument channel of an endoscope. The treatment instruments have different outer diameter dimensions depending on their types. The adapters are attached to the treatment instrument inserting sections having different outer diameter dimensions to form surface to be pressed which has a fixed dimension for the treatment instruments. The adapters respectively have a through-hole corresponding to each diameter dimension of the treatment instrument inserting sections. The advancing and retracting device includes a pair of rollers separated by a distance corresponding to the surface to be pressed of a fixed dimension. The rollers hold the surface to be pressed of the adapter attached to the treatment instrument inserting section under a predetermined pressure. A rotation of the rollers with the adapter being held between the rollers causes the adapter to advance and retract.

## Description

### BACKGROUND OF THE INVENTION

### Field of the Invention

The present invention relates to an endoscope treatment system including an endoscope and a treatment instrument, in which the treatment instrument is automatically inserted into and withdrawn out of a treatment instrument channel provided in the endoscope.

### Description of Related Art

In recent years, an endoscope is widely used in the medical field. An endoscope has an elongated inserting section and an operating section provided at the base end of the inserting section. Generally, the elongated inserting section has a tip end to which a bendable bending section is provided. The operating section is provided with a knob for operating the bending section to bend accordingly, various switches for functioning various operations of the endoscope, and the like.

An endoscope in the medical field has an inserting section which is inserted into a body cavity of a subject for observation of organs in the body cavity. In such an endoscope, the inserting section is provided with a treatment instrument channel through which a treatment instrument is introduced into the body cavity for various procedures and checks.

In order to insert the treatment instrument into the treatment instrument channel of an endoscope, an operator holds a sheath which forms an inserting section of the treatment instrument by hand to manually insert the sheath into the treatment instrument channel. However, the manual insertion is inconvenient.

To address the inconvenience, for example, Japanese Patent Laid-Open No. 9-492 discloses an inserting and withdrawing apparatus for treatment instrument of endoscope in which a treatment instrument is automatically inserted into and withdrawn out of a forceps channel of an endoscope. The inserting and withdrawing apparatus for treatment instrument of endoscope includes a rotatable shaft which projects outward from a body of the apparatus, and the rotatable shaft is configured so that a plurality of types of rotating rollers having different outer diameters can selectively attached, thereby a treatment instrument having a different outer diameter can be automatically inserted into and withdrawn out of the forceps channel of the endoscope. In the configuration, a treatment instrument having any outer diameter can be inserted into and withdrawn out of the forceps channel of the endoscope at a predetermined speed by exchanging the rotating rollers in accordance with the outer diameter of the treatment instrument.

However, medical staffs need to prepare rotating rollers having an outer diameter dimension appropriate to a treatment instrument to be used in a procedure or check in advance. Also when a plurality of treatment instruments are to be exchanged during a procedure or check, a task to exchange rotating rollers in accordance with treatment instruments to be used in the operation is required as well as the task to prepare the treatment instruments and rotating rollers for the treatment instruments, which may be troublesome.

A first object of the present invention is to provide an endoscope treatment system including one electric advancing and retracting device and a plurality of types of treatment instruments having sheaths of different outer diameter dimensions, in which any adjustment such as a change of a space between rollers of the electric advancing and retracting device is eliminated, which reduces staffs workload in a surgical operation, and the shortened period of time for procedure reduces operator's workload and patient's discomfort.

A second object of the present invention is to provide an endoscope treatment system in which the sheath is caused to be advanced and retracted at a fixed speed by a rotation of the rollers of the electric advancing and retracting device independently of the types of treatment instrument.

A third object of the present invention is to provide an endoscope treatment system in which an adapter and the sheath can integrally move as a unit when a rotation of the rollers of the electric advancing and retracting device causes the sheath to advance and retract.

### SUMMARY OF THE INVENTION

An endoscope system according to the present invention comprises:
a plurality of types of treatment instruments having treatment instrument inserting sections which can be inserted into the treatment instrument channel of an endoscope and have different outer diameter dimensions;
a plurality of types of adapters including surfaces to be pressed which have a fixed dimension when the adapters are attached to the treatment instrument inserting sections of the treatment instruments, and through-holes formed therein through which the treatment instrument inserting sections having different outer diameter dimensions are individually inserted; and
an advancing and retracting device which includes a pair of rollers which are separated by a predetermined distance, and holds the surface to be pressed of the adapter attached to the treatment instrument inserting section under a predetermined pressure between the rollers, so that, with the adapter being held, a rotation of the rollers causes the adapter to advance and retract.

The above and other objects, features and advantages of the invention will become more clearly understood from the following description referring to the accompanying drawings.

### BRIEF DESCRIPTION OF THE DRAWINGS

FIG. 1 is a view illustrating an endoscope treatment system which includes adapters having a fixed diameter dimension to be attached to sheaths having different diameter dimensions;
FIG. 2A is a view illustrating a circumferential projection on a biopsy forceps, and a circumferential groove in an adapter;
FIG. 2B is a view illustrating a circumferential projection on a high-frequency snare, and a circumferential groove in an adapter;
FIG. 2C is a view illustrating a circumferential projection on a basket forceps, and a circumferential groove in an adapter;
FIG. 3 is a longitudinal cross sectional view illustrating a relationship between a pair of rollers mounted to an electric advancing and retracting device and an adapter attached to a sheath;
FIG. 4 is a lateral cross sectional view illustrating a relationship between a pair of rollers mounted to an electric advancing and retracting device and an adapter attached to a sheath;
FIG. 5 is a view illustrating an adapter attached to a sheath which is held between a pair of rollers;
FIG. 6 is a view illustrating an adapter having a tip end surface from which a sheath of a treatment instrument is projected by a predetermined dimension;
FIG. 7 is a view illustrating a configuration of an endoscope treatment system which includes a sheath having a fixed diameter dimension independently of the types of treatment instruments;
FIG. 8 is a view illustrating a relationship between a pair of rollers mounted to an electric advancing and retracting device and a sheath having a fixed diameter dimension;
FIG. 9 is a view illustrating a configuration of a treatment instrument which includes sheath portions having different diameter dimensions;
FIG. 10A is a view illustrating a relationship between a sheath having an elliptical cross section and having a dimension X along its major axis and a pair of rollers; and
FIG. 10B is a view illustrating a relationship between a sheath having an elliptical cross section and having a dimension Y along its major axis and a pair of rollers.

### DETAILED DESCRIPTION OF PREFERRED EMBODIMENTS

Now, several embodiments of the present invention will be explained below with reference to accompanying drawings.

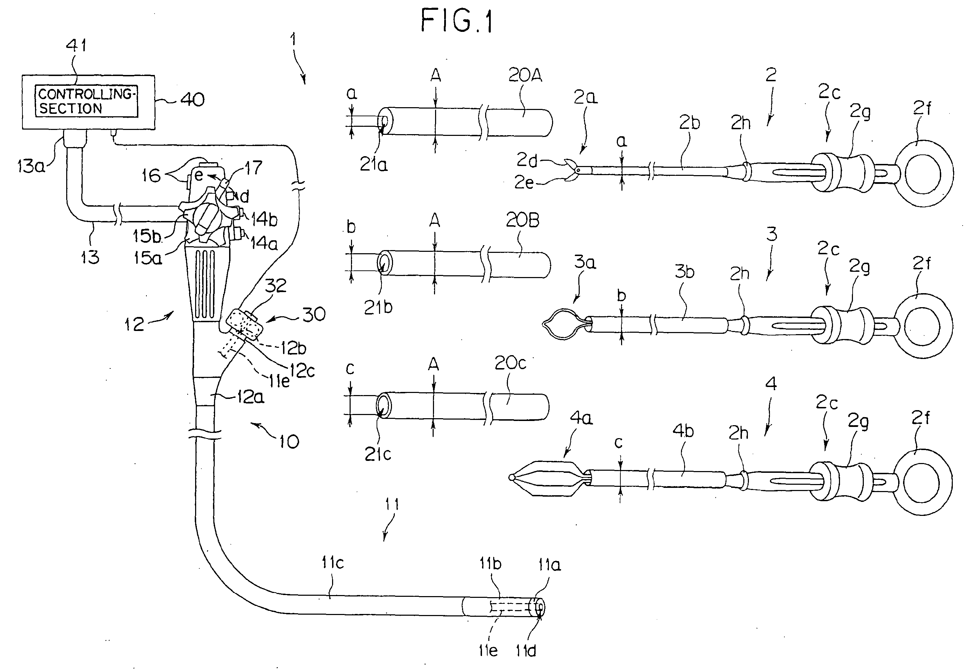

Referring to FIGS. 1 to 6, a first embodiment of the present invention will be explained.

As shown in FIG. 1, an endoscope system 1 generally includes an endoscope 10, a plurality of types of treatment instruments 2, 3, 4 ..., an adapter to form a treatment instrument inserting section 20 which is attached to the treatment instrument inserting section of the treatment instruments 2, 3, 4 ... (hereinafter, briefly referred to as an adapter), an electric device for advancing and retracting a treatment instrument inserting section 30 which is an electrically operated advancing and retracting device (hereinafter, referred to as electric advancing and retracting device), and a controlling device 40 having a controlling section 41.

The controlling device 40 also serves as a light source and a video processor, and includes a controlling section 41 therein. The controlling device 40 is adopted to be connected with a displaying apparatus such as a liquid crystal display (not shown) for displaying an endoscope image.

In this embodiment, the treatment instruments include a biopsy forceps 2, a high-frequency snare 3, and a basket forceps 4.

The biopsy forceps 2 comprises, in order from a tip end thereof, a tissue sampling section 2a which is a function part, an elongated sheath 2b which is a treatment instrument inserting section, a handle section 2c which is an operating part. The tissue sampling section 2a is provided at a tip end of the sheath 2b. The tissue sampling section 2a includes a pair of biopsy cups 2d and 2e which are rotatably configured. The sheath 2b of the biopsy forceps 2 has an outer diameter dimension a. Through the sheath 2b, an operation wire (not shown) is inserted.

The handle section 2c includes a finger engaging ring 2f and slider 2g. The finger engaging ring 2f has a hole formed therein to which an operator's thumb for example may be engaged. The slider 2g has a pair of flanges between which an operator's second and third fingers may be engaged. The handle section 2c has a circumferential projection 2h which forms a removable section, at tip end of the handle section nearer.

The operation wire is controlled to be advanced or retracted by an operation of the handle section 2c. Specifically, an advancement and retraction of the operation wire corresponding to an operation of the slider 2g along an axis of the handle section 2c causes the cups 2d and 2e of the tissue sampling section 2a to move to its closed position and its opened position.

The high-frequency snare 3 comprises, in order from a tip end thereof, a snare section 3a which is a function part, an elongated sheath 3b which is a treatment instrument inserting section, and a handle section 2c. The snare section 3a is projectably and retractably provided relative to a tip end of the sheath 3b. The sheath 3b of the high-frequency snare 3 has an outer diameter dimension b. Through the sheath 3b, an operation wire (not shown) is inserted.

In the high-frequency snare 3, an advancement of the slider 2g causes the snare section 3a to be extracted from the tip end of the sheath 3b to form a loop. To the contrary, a retraction of the slider 2g when the loop is formed causes the loop of the snare section 3a to be contained in the sheath 3b.

To the slider 2g of the high-frequency snare 3 in this embodiment, a high frequency wiring cord (not shown) is electrically connected at one end thereof. The high frequency wiring cord is connected to a high frequency power source (not shown) at the other end thereof. The high frequency wiring cord is connected to a metallic operation wire (not shown) provided in the sheath 3b via the slider 2g to be electrically connected to the snare section 3a.

The basket forceps 4 comprises, in order from a tip end thereof, a stone basket 4a which is a function part, an elongated sheath 4b which is a treatment instrument inserting section, and a handle section 2c. The stone basket 4a is provided at a tip end of the sheath 4b. The sheath 4b of the basket forceps 4 has an outer diameter dimension c. Through the sheath 4b, an operation wire (not shown) is inserted.

In the basket forceps 4, an advancement and retraction of the slider 2g causes the stone basket 4a to continuously move to its closed position to grasp stones and its opened position to extract stones.

It should be noted that the treatment instruments used in the endoscope system of the present invention are not limited to the above biopsy forceps 2, the high-frequency snare 3, and the basket forceps 4, and may be a contrast tube, a high-frequency knife, and the like.

The adapter 20 is means for adjusting a pressing force, and has a pipe-like shape having a longitudinal through-hole (hereinafter, briefly referred to as through-hole) 21 formed therein. The adapter 20 may have a circular cross section, and has an outer diameter dimension A. The outer diameter dimension A of the adapter 20 is set in consideration of a space B between roller surfaces of the rollers for an electric advancing and retracting device 30 which will be explained below. In other words, the adapter 20 having the outer diameter dimension A can be placed between the rollers which are separated by a space B so that the outer surface of the adapter 20 is pressed under pressure by the rollers, thereby an optimal holding pressure for an advancement and retraction of the adapter 20 works on the adapter. This means the outer surface of the adapter 20 is a surface to be pressed.

The adapters 20 may include a plurality of types depending on a diameter dimension of through-hole. Specifically, an adapter 20A has a through-hole 21a having a diameter dimension a. An adapter 20B has a through-hole 21 b having a diameter dimension b. An adapter 20C has a through-hole 21c having a diameter dimension c. The adapters 20 are made of a resin material having a flexibility such as polyethylene, polypropylene, fluorocarbon resin, PEEK, EVA, nylon, polyurethane, thermoplastic elastomer, and the like, and has an outer surface having a fixed surface roughness, independently of the types of the adapters.

Thus, a rotation of the rollers of the advancing and retracting device 30 causes any one of adapters 20A, 20B, and 20C to advance and retract in a similar way independently of the types of the adapters 20. It should be noted that the adapters 20 may have any configuration other than that of a resin tube, and may be a resin tube containing a metal mesh and coil, or a two-layer tube including a resin inner layer and a metal outer layer.

The adapter 20A is attached to the sheath 2b of the biopsy forceps 2 to form an inserting section 22 for advancing and retracting the biopsy forceps 2. The adapter 20B is attached to the sheath 3b of the high-frequency snare 3 to form an inserting section 22 for advancing and retracting the high-frequency snare 3. The adapter 20C is attached to the sheath 4b of the basket forceps 4 to form an inserting section 22 for advancing and retracting the basket forceps 4. That is, the attachment of the adapter 20A to the sheath 2b of the biopsy forceps 2, the attachment of the adapter 20B to the sheath 3b of the high-frequency snare 3, and the attachment of the adapter 20C to the sheath 4b of the basket forceps 4 make each of the biopsy forceps 2, the high-frequency snare 3, and the basket forceps 4 have the inserting section 22 for advancement and retraction which has a fixed diameter dimension A.

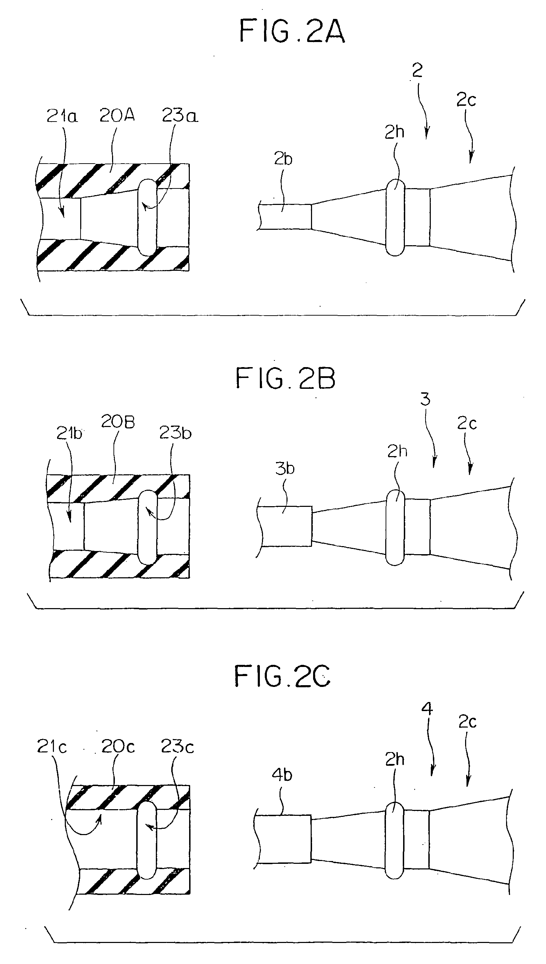

The through-hole 21 of the adapter 20 has an inner surface, and as shown in FIGS. 2A to 2C, circumferential grooves 23a, 23b, and 23c are formed in the inner surface near a base end of the through-hole 21, in which the circumferential projection 2h is engagedly fitted to form a removable section. Therefore, when the sheaths 2b, 3b, and 4b of the treatment instruments are inserted into the through-holes 2 a, 21 b, and 21c of the adapters 20A, 20B, and 20C, the adapters 20A, 20B, and 20C are integrally and securely attached to the sheaths 2b, 3b, and 4b by engagedly fitting the circumferential projection 2h in the circumferential grooves 23a, 23b, and 23c.

A height dimension and a width dimension of the circumferential projection 2h, and a width dimension and a depth dimension of the circumferential grooves 23a, 23b, and 23c are individually set in consideration of a locking power. A specific locking power is set higher than a holding pressure by the rollers. This enables the adapter and the sheath to advance and retract together as a unit upon a rotation of rollers when the adapter attached to the sheath is placed between the rollers. In addition, in order to improve the insertion of the treatment instrument sheath through the adapter 20, the inner surface of the adapter 20 may be provided with a lubricant coating.

The endoscope 10 comprises an inserting section 11, an operating section 12, and a universal cord 13. The operating section 12 also serves as a holding section, and is provided at a base end of the inserting section 11. The universal cord 13 extends from a side of the operating section 12 to be connected to the controlling device 40 via a connector 13a which is provided at a base end of the universal cord.

The inserting section 11 comprises, in order from a tip end thereof, a rigid tip end section 11a, a bendable bending section 11b, and a flexible tube section 11c having a flexibility, which are all connected to each other. The operating section 12 is provided with an anti-break fixing section 12a which is connected to a base end of the flexible tube section 11c. The operating section 12 includes an air/water supplying button 14a for supplying air/water, a suction button 14b for suctioning, bending control knobs 15a and 15b for controlling the bending of the bending section 11b, various switches 16 for controlling endoscope images which are captured by an image capturing means such as a CCD mounted to the tip end 11 a and displayed on a screen of a displaying apparatus, an operation lever 17 to give an instruction on a rotation of the electric advancing and retracting device 30, and the like. The operation lever 17 is rotatable by a predetermined angle in the directions shown by an arrow d and an arrow e. It should be noted that in this embodiment, the operating section 12 comprises the operation lever 17, but the operation lever 17 may be a separate element from the endoscope 10.

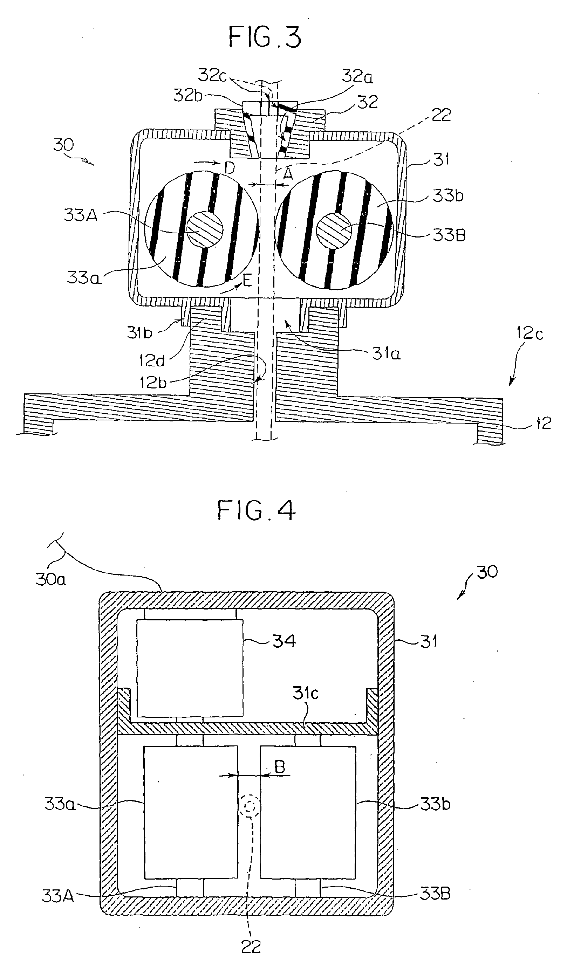

The operation lever 17 may be a return to zero switch. Specifically, the operation lever 17 outputs an instruction signal for advancement of sheath (hereinafter, briefly referred to as advancement signal) which causes the roller 33a to rotate in a direction D in FIG. 3 to the controlling device 40 when the operation lever 17 is pushed in a direction shown by an arrow d of FIG. 1. To the contrary, the operation lever 17 outputs an instruction signal for retraction of sheath (hereinafter, briefly referred to as retraction signal) which causes the roller 33a to rotate in a direction E in FIG. 3 to the controlling device 40 when the operation lever 17 is pushed in a direction shown by an arrow e in FIG. 1.

The endoscope 10 comprises a treatment instrument channel 11e which is in communication between an opening for treatment instrument 12b and a tip end opening 11d at the tip end section 11a. Through the treatment instrument channel 11e, the tissue sampling section 2a and sheath 2b of the biopsy forceps 2, the snare section 3a and sheath 3b of the high frequency snare 3, and the stone basket 4a and sheath 4b of the basket forceps 4, and the like are inserted. Also, through the treatment instrument channel 11e, the adapter 20A attached to the tissue sampling section 2a and sheath 2b of the biopsy forceps 2, the adapter 20B attached to the snare section 3a and sheath 3b of the high frequency snare 3, and the adapter 20C attached to the stone basket 4a and sheath 4b of the basket forceps 4 are inserted. This means the treatment instrument channel 11e has an inner diameter dimension which is larger than the outer diameter dimension A of the adapters 20A, 20B, and 20C.

With reference to FIG. 1, FIG. 3, and FIG. 4, the electric advancing and retracting device 30 will be explained below.

The electric advancing and retracting device 30 comprises has a housing 31 which is provided with two rotatable rollers 33a and 33b therein. The housing 31 includes opposite sides, and one of the sides has a sheath inserting section 32 through which the treatment instrument inserting section of a treatment instrument is inserted. The sheath inserting section 32 has a communicating hole 32a formed therein into which a forceps plug 32b of an elastic material is placed. The forceps plug 32b has a slit 32c formed therein.

Through the slit 32c, the adapter 20A attached to the sheath 2b of the biopsy forceps 2, the adapter 20B attached to the sheath 3b of the high-frequency snare 3, and the adapter 20C attached to the sheath 4b of the basket forceps 4, and the like are inserted. That is, the slit 32c is configured to allow the inserting section 22 for advancement and retraction to be inserted.

The other of the sides of the housing 31 has an inserting section inserting hole 31 a formed therein through which the adapters 20A, 20B, and 20C pass via the slit 32c. Surrounded the inserting section inserting hole 31 a is a scope fixing section 31 b for removably coupling the housing 31 to a projection 12d which forms a treatment instrument mounting section 12c. The scope fixing section 31 b is configured to be hermetically connected to the treatment instrument mounting section 12c of the operating section 12.

The two rollers 33a and 33b in the housing 31 are individually made of a resin material having an elasticity. The rollers 33a and 33b are integrally fixed to rotation shafts 33A and 33B thereof respectively. The rotation shaft 33A is a driving shaft, and an operation of a motor 34 which is a mounted in the housing 31 causes the rotation shaft 33A to be rotated. To the contrary, the rotation shaft 33B is a driven shaft which is rotatably mounted in the housing 31. An electrical cable 30a extends from the electric advancing and retracting device 30 and is removably connected to the controlling device 40.

The rotation shaft 33A and the rotation shaft 33B are rotatably supported by side walls and a supporting plate 31c of the housing 31 so that the rotation shaft 33A and the rotation shaft 33B are parallel to each other and a space between the roller surfaces of the rollers 33a and 33b which are fixedly mounted to the rotation shafts 33A and 33B respectively is separated by a predetermined space B. The space B between the roller surface of the roller 33a and the roller surface of the roller 33b are set to be smaller than the outer diameter dimension A of the adapter 20 in advance. This makes the outer surfaces of the adapters 20A, 20B, and 20C are held under pressure between the roller surface of the roller 33a and the roller surface of the roller 33b when the adapters 20A, 20B, and 20C are inserted through the slit 32c.

The motor 34 is controlled to be driven by an advancement signal or retraction signal which is output in response to a pushing operation of the operation lever 17 in the direction of the arrow d or the direction of the arrow e. That is, as shown by dotted line for example, with the adapter 20A attached to the sheath 2b being held between the rollers 33a and 33b, a rotation of the motor 34 causes the roller 33a to rotate, which makes the adapter 20A and the sheath 2b advance and retract together as a unit.

It should be noted that, in this embodiment, the advancing and retracting device for advancing and retracting a treatment instrument inserting section of a treatment instrument is an electrically operated advancing and retracting device. However, the advancing and retracting device is not limited to the electrically operated one, and may be a device which is manually operated to advance and retract a sheath.

Operations of the endoscope system 1 configured in the way described above will be more specifically explained below.

In using the endoscope system 1 in a medical surgery, a staff confirms if an operator uses the electric advancing and retracting device 30 or not. When the staff confirms that the operator uses the electric advancing and retracting device 30, the staff prepares a treatment instrument and also an adapter appropriate to the treatment instrument to be used in the medical surgery with the electric advancing and retracting device 30. Then the electric advancing and retracting device 30 is mounted to the treatment instrument mounting section 12c of the endoscope 10. Also, the treatment instruments designated by the operator, such as a biopsy forceps 2 and a high-frequency snare 3, are prepared as well as the adapters 20A and 20B by a staff.

A staff attaches the adapter 20A to a sheath 2b of the biopsy forceps 2 to form an adapter integrated biopsy forceps (hereinafter, briefly referred to as an integrated biopsy forceps) 2A, and attaches the adapter 20B to a sheath 3b of the high-frequency snare 3 to form an adapter integrated high-frequency snare (not shown, hereinafter, briefly referred to as an integrated high-frequency snare). These attachments make the treatment instrument inserting section of the biopsy forceps 2 with the sheath 2b of the diameter dimension a and the treatment instrument inserting section of the high-frequency snare 3 with the sheath 3b of the diameter dimension b have the fixed diameter dimension A of the adapters 20A, 20B. In other words, the attachment of the adapter to the sheath of the treatment instrument allows any treatment instrument such as the integrated biopsy forceps 2A, the integrated high-frequency snare, and the like to have an inserting section 22 for advancement and retraction having the outer diameter dimension A.

The operator inserts the inserting section 11 of the endoscope 10 toward a target site in a body cavity of a subject while observing an endoscope image. At this time, the operator performs bending operation on the bending section 1 16 as well as inserting operation of the inserting section, while checking an endoscope image which is displayed on a screen of a displaying apparatus.

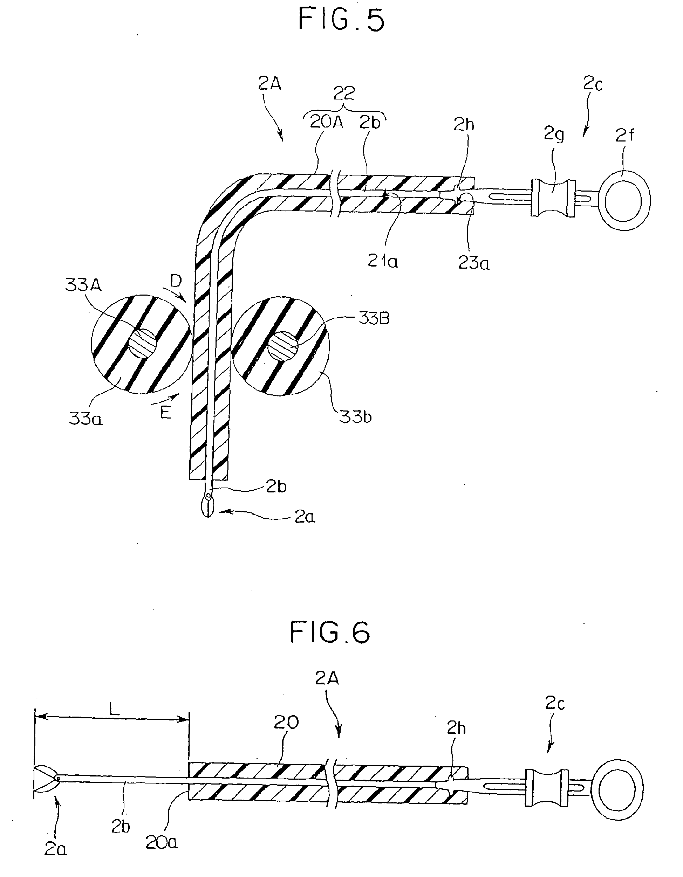

If the operator samples a tissue, the operator bends the tip end 11a of the insertion section 11 to face to the tissue at the target site for an easier sampling. Meanwhile, a staff inserts the inserting section 22 for advancement and retraction of the integrated biopsy forceps 2A through the slit 32c in the forceps plug 32b into the housing 31 of the electric advancing and retracting device 30. As a result, as shown in FIG. 5, the outer surface of the adapter 20A which is the outer surface of the inserting section 22 for advancement and retraction is held under pressure between the roller surfaces of the two rollers 33a and 33b.

At this point, the operator pushes the operation lever 17 into the direction of the arrow d in FIG. 1 while observing an endoscope image. In response to the manual operation of the lever by the operator, an advancement signal is output to the controlling device 40, and the controlling section 41 outputs a control signal which causes the roller 33a to start to rotate in the direction of the arrow D.

With the rotation, the inserting section 22 for advancement and retraction of the sheath 2b and the adapter 20A passes through the inserting section inserting hole 31 a and the opening for treatment instrument 12b, and is inserted in the treatment instrument channel 11e to advance toward the tip end opening 11d. Then the tissue sampling section 2a at the tip end of the sheath 2b is extracted from the tip end opening 11d into the body cavity.

After the extraction, the operator operates the operation lever 17 as needed, and a staff operates the handle section 2c as needed based on instructions by the operator to perform a tissue sampling with the tissue sampling section 2a. After the tissue sampling, the operator pushes the operation lever 17 into the direction of the arrow e. In response to the manual operation of the lever by the operator, a retraction signal is output to the controlling device 40, and the controlling section 41 outputs a control signal which causes the roller 33a to rotate in the direction of the arrow E.

With the rotation, the tissue sampling section 2a extracted into the body cavity is retracted through the treatment instrument channel 11e. The operator or a staff collects the tissue sampled by the tissue sampling section 2a after the inserting section 22 for advancement and retraction is released from the holding between the rollers 33a and 33b.

If the operator subsequently removes a part of lesion with the high-frequency snare 3, a staff inserts the inserting section 22 for advancement and retraction of the integrated high-frequency snare through the slit 32c in the forceps plug 32b into the housing 31 of the electric advancing and retracting device 30. Also, the integrated high-frequency snare has the inserting section 22 for advancement and retraction, so that the outer surface of the adapter 20B which is the outer surface of the inserting section 22 for advancement and retraction is held under pressure between the roller surfaces of the two rollers 33a and 33b, as in the biopsy forceps 2 having the sheath 2a to which the adapter 20A shown in FIG. 5 is attached. This configuration allows the inserting section 22 for advancement and retraction having the sheath 3b and adapter 20B of the integrated high-frequency snare A to advance and retract in the same way as the inserting section 22 for advancement and retraction of the integrated biopsy forceps 2A.

That is, inserting sections for advancement and retraction of various treatment instruments can be held under pressure between the rollers 33a and 33b in the electric advancing and retracting device 30 in the same way independently of the types of the treatment instruments, without any adjustment such as a change of the space B between the rollers 33a and 33b. This allows an adapter attached to a sheath of a treatment instrument having any diameter dimension to advance and retract in the same way by an electric advancing and retracting device. Thus, the operating section moves at a fixed speed independently of the types of treatment instruments.

In this way, an endoscope system of the present invention comprises a plurality of types of treatment instruments having sheaths of different outer diameter dimensions which are to be inserted into a treatment instrument channel of an endoscope, and an electric advancing and retracting device to advance and retract the sheaths of the treatment instruments, In the system, an adapter is provided for each treatment instrument, which has an inner diameter dimension corresponding to an outer diameter dimension of a sheath of the treatment instrument, and has an outer diameter corresponding to a space between rollers which form an electric advancing and retracting device. Each adapter is attached to a sheath of a corresponding treatment instrument before the endoscope system is used. This makes the portions corresponding to a sheath of treatment instruments to be used in the endoscope system have the same fixed outer diameter dimension of the adapter. So, each sheath of the treatment instruments to which the adapter is attached can be held under pressure between the rollers of the electric advancing and retracting device in the same way independently of the types of treatment instruments.

Therefore, in the endoscope system including one type of electric advancing and retracting device and, a plurality of types of treatment instruments having sheaths of different outer diameter dimensions, any adjustment such as a change of the space between the rollers of the electric advancing and retracting device is eliminated, which reduces staff's workload in a surgical operation, and the shortened period of time for procedure reduces operator's workload and patient's discomfort.

In addition, the adapter to be attached to sheaths having various diameters is configured to have an outer diameter dimension corresponding to a space between rollers, to be made of a resin material having a flexibility, and to include an outer surface having a fixed surface roughness. So, as for a treatment instruments having a sheath to which the adapter is attached, the sheath is caused to be advanced and retracted at a fixed speed by a rotation of the rollers of the electric advancing and retracting device, independently of the types of treatment instruments.

In addition, the adapter to be attached to sheaths having various diameters has a through-hole, and in an inner surface of the through-hole at the base end thereof, a circumferential groove is formed. When the adapter is attached to a sheath, a circumferential projection formed on a handle section of a treatment instrument is engagedly fitted into the circumferential groove in the sheath so that the sheath and the adapter are integrally attached to each other. The locking power in the engagedly fitting of the circumferential projection into the circumferential groove is set to be larger than the holding power of the rollers. Thus, the adapter and the sheath can integrally move as a unit when a rotation of the rollers of the electric advancing and retracting device causes the sheath to advance and retract.

In the above described embodiment as shown in FIG. 5, the adapter 20A is configured to cover the generally entire length of the sheath 2b. However, the adapter is not limited to be configured to cover the generally entire length of a sheath. In other words, as shown an integrated biopsy forceps 2A in FIG. 6, for example, the adapter 20 attached to the sheath 2b may be configured to have a length dimension in advance so that the tissue sampling section 2a, which is a functioning part, can be projected out of the tip end surface 20a of the adapter 20 by a predetermined length dimension L.

In this way, by setting the length dimension of the adapter in consideration of the length dimension L, the tip end of the adapter which has a diameter dimension larger than the outer diameter dimension of sheath can be placed at a desired position in the treatment instrument channel through a treatment instrument opening, for example in order to prevent any damage on the flexibility of the inserting section of the endoscope.

The adapter may have an elliptical cross section or a flattened cross section other than a circular cross section.

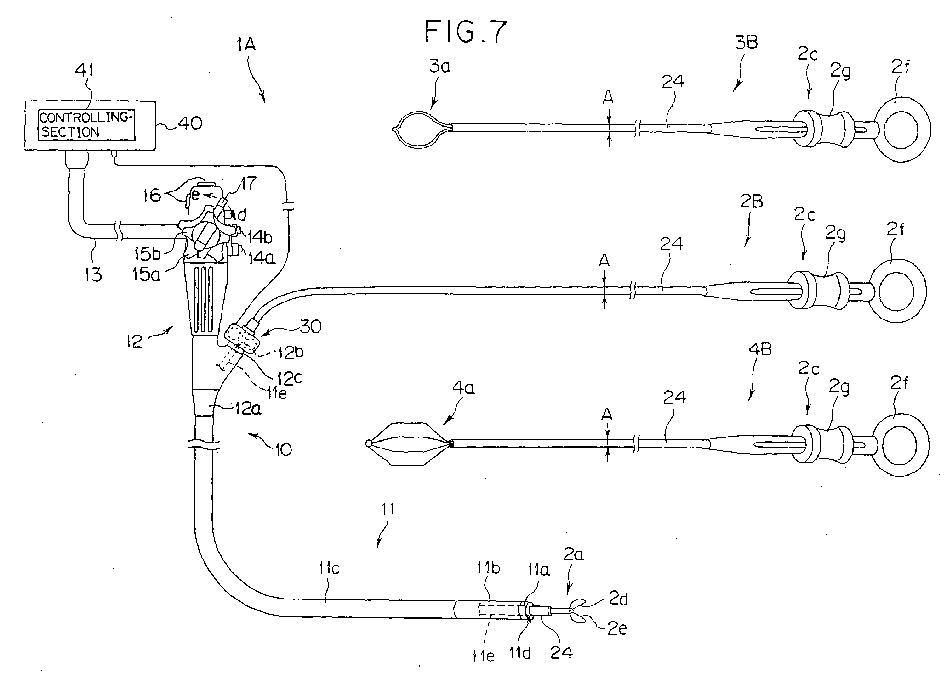

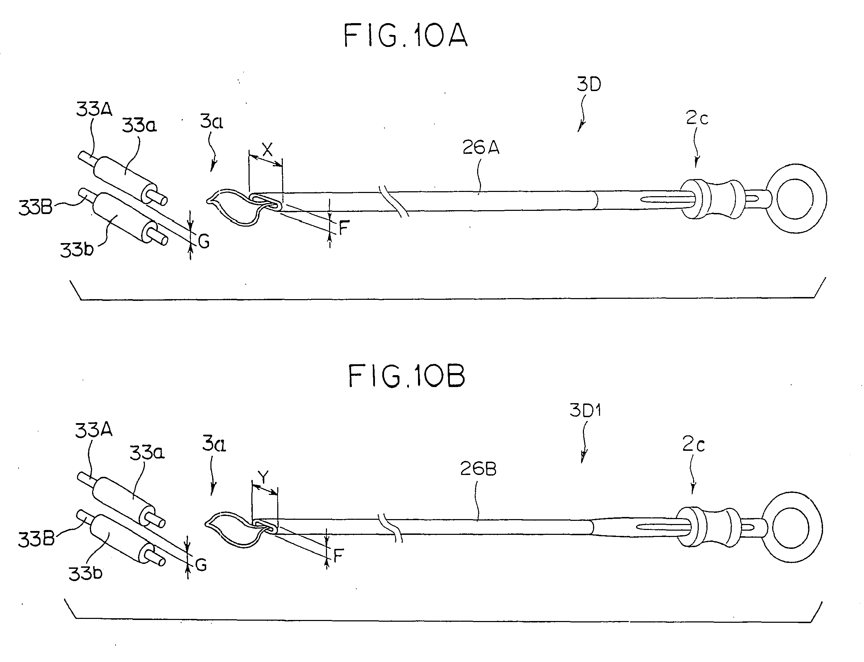

Referring to FIGS. 7 to 10B, a second embodiment of the present invention will be explained.

As shown in FIG. 7, an endoscope system 1A generally includes an endoscope 10, a biopsy forceps 2B, a high frequency snare 3B, a basket forceps 4B, which are a plurality of types of treatment instruments, an electric advancing and retracting device 30, and a controlling device 40.

The biopsy forceps 2B includes, in order from a tip end thereof, a tissue sampling section 2a which is a functioning section, a sheath 24 which is a treatment instrument inserting section, that is, a pressing force adjusting means, and a handle section 2c which is an operating section. The sheath 24 has a tip end at which a tissue sampling section 2a is provided. The sheath 24 of the biopsy forceps 2B has an outer diameter dimension A which is set in consideration of the space B between the rollers 33a and 33b of the electric advancing and retracting device 30. That is, the outer surface of the sheath 24 is a surface to be pressed between the roller surfaces under pressure. The high-frequency snare 3B includes, in order from a tip end thereof, a snare section 3a which is a functioning section, a sheath 24, and a handle section 2c. The basket forceps 4 includes, in order from a tip end thereof, a stone basket 4a which is a functioning section, a sheath 24, and a handle section 2c.

This means each of the biopsy forceps 2B, the high-frequency snare 3B, and the basket forceps 4B has a sheath 24 having an outer diameter dimension A. The sheath 24 is made of a resin material having a flexibility, and has an outer surface having a fixed surface roughness for all of the biopsy forceps 2B, the high-frequency snare 3B, and the basket forceps 4B.

It should be noted that in the endoscope system I A, the treatment instruments are the biopsy forceps 2B, the high-frequency snare 3B, and the basket forceps 4B. However, in the endoscope system 1A, the treatment instruments are not limited to those, and may be a cannula, a high frequency knife, and the like. The cannula, the high frequency knife, and the like also include the sheath 24 having the outer diameter dimension A. In the treatment instruments of this embodiment, the circumferential projection which is engagedly fitted into the circumferential groove formed in the adapter shown in the first embodiment is not required. So, the handle section 2c does not include a circumferential projection. In addition, the sheath 24 may have a through-hole formed therein which has a different diameter for each treatment instrument, or has a same diameter independently of the types of the treatment instruments.

Other configuration of the endoscope system 1A is similar to that in the first embodiment, and members which are the same or similar to those in the first embodiment are designated with like reference numerals, and will not be explained in detail below.

Now, operations of the endoscope system 1A configured in the way described above will be specifically explained below.

In using the endoscope system 1A in a medical surgery, a staff confirms if an operator uses the electric advancing and retracting device 30 or not. When the staff confirms that the operator uses the electric advancing and retracting device 30, the staff prepares the electric advancing and retracting device 30 and also treatment instruments such as the biopsy forceps 2B and the high-frequency snare 3B to be used in the medical surgery. Then the electric advancing and retracting device 30 is mounted to the treatment instrument mounting section 12c of the endoscope 10 by a staff.

The operator inserts the inserting section 1 1 of the endoscope 10 toward a target site in a body cavity of a subject while observing an endoscope image. At this time, the operator performs bending operation on the bending section 11 b as well as inserting operation of the inserting section, while checking an endoscope image which is displayed on a screen of a displaying apparatus.

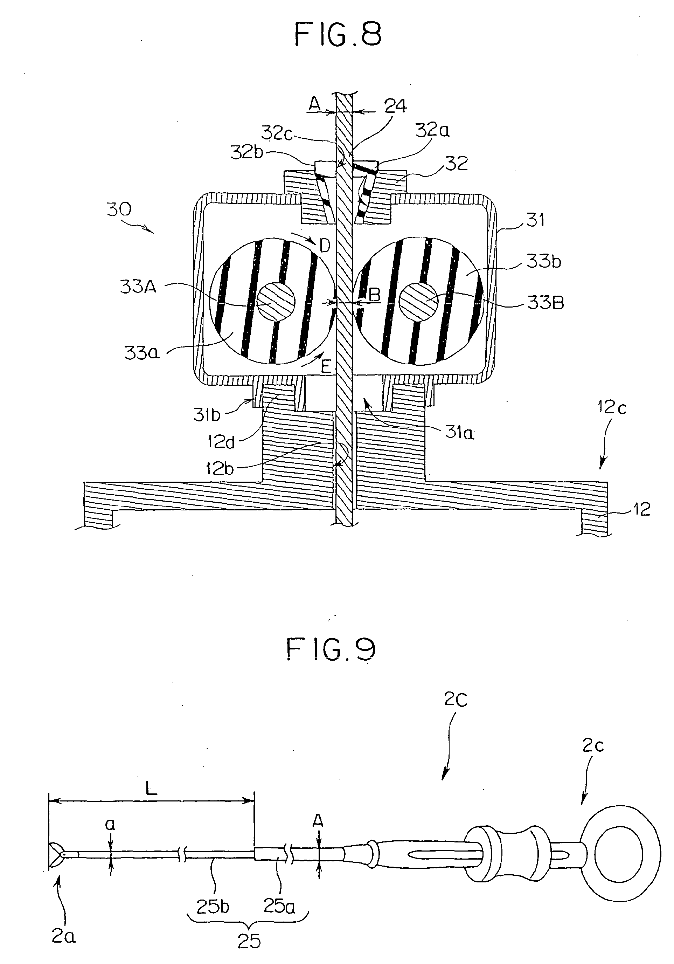

If the operator samples a tissue with the biopsy forceps 2B, the operator bends the tip end 11a of the insertion section 11 to face to the tissue at the target site for an easier sampling. Meanwhile, a staff inserts the sheath 24 of the biopsy forceps 2B through the slit 32c in the forceps plug 32b into the housing 31 of the electric advancing and retracting device 30. As a result, as shown in FIG. 8, the outer surface of the sheath 24 is held under pressure between the roller surfaces of the two rollers 33a and 33b.

At this point, the operator pushes the operation lever 17 into the direction of the arrow d in FIG. 1 while observing an endoscope image. In response to the manual operation of the lever by the operator, an advancement signal is output to the controlling device, and the controlling section 41 outputs a control signal which causes the roller 33a to start to rotate in the direction of the arrow D.

With the rotation, the sheath 24 passes through the inserting section inserting hole 31 a and the opening for treatment instrument 12b, and is inserted in the treatment instrument channel 11e to advance toward the tip end opening 11d. Then the tissue sampling section 2a at the tip end of the sheath 24 is extracted from the tip end opening 11d into the body cavity.

After the extraction, the operator operates the operation lever 17 as needed, and a staff operates the handle section 20 as needed based on instructions by the operator to perform a tissue sampling with the tissue sampling section 2a. After the tissue sampling, the operator pushes the operation lever 17 into the direction of the arrow e. In response to the manual operation of the lever by the operator, a retraction signal is output to the controlling device, and the controlling section 41 outputs a control signal which causes the roller 33a to rotate in the direction of the arrow E.

With the rotation, the tissue sampling section 2a extracted into the body cavity is retracted through the treatment instrument channel 11e. The operator or a staff collects the tissue sampled by the tissue sampling section 2a after the sheath 24 is released from the holding between the rollers 33a and 33b.

If the operator subsequently removes a part of lesion with the high-frequency snare 3B, a staff inserts the sheath 24 of the high-frequency snare 3B through the slit 32c in the forceps plug 32b into the housing 31 of the electric advancing and retracting device 30. In the case of the high-frequency snare 3B also, the outer surface of sheath 24 is held under pressure between the roller surfaces of the two rollers 33a and 33b, as in the case of the biopsy forceps 2B shown in FIG. 8. This configuration allows the sheath 24 of the high-frequency snare 3B to advance and retract in the same way as the sheath 24 of the biopsy forceps 2B.

In this way, sheaths having a same diameter for various treatment instruments can be disposed with being held under pressure between the rollers 33a and 33b of the electric advancing and retracting device 30 in the same way independently of the type of the treatment instruments, without any adjustment such as a change of the space B between the rollers 33a and 33b and the like. This allows the sheath to be advanced and retracted in the same way by the electric advancing and retracting device independently of the type of the treatment instruments. Thus, the functioning section moves at a fixed speed independently of the types of treatment instrument.

Therefore, the endoscope system comprises a plurality of types of treatment instruments having sheaths having a fixed outer diameter dimension to be inserted into a treatment instrument channel of an endoscope, and an electric advancing and retracting device to advance and retract the sheaths of the treatment instruments. So, each sheath of the treatment instruments can be disposed with being held under pressure between the rollers of the electric advancing and retracting device in the same way independently of the types of treatment instrument.

Therefore, in the endoscope system including one electric advancing and retracting device and a plurality of types of treatment instruments, any adjustment such as a change of the space between the rollers of the electric advancing and retracting device and the like is eliminated, which significantly reduces staffs workload in a surgical operation.

The sheaths which are configured to have a fixed diameter dimension are made of a resin material having a flexibility, and include an outer surface having a fixed surface roughness. So, the sheath is caused to be advanced and retracted at a fixed speed by a rotation of the rollers of the electric advancing and retracting device independently of the types of treatment instrument.

It should be noted that, in the second embodiment, the sheath is configured to have the outer diameter dimension A along its entire longitudinal length. However, the sheath is not limited to be configured to have the outer diameter dimension A along its entire longitudinal length. Specifically, for example in determining the entire longitudinal length of the sheath, the sheath may be configured to include a sheath, nearer to the tip end of a sheath having a diameter dimension A as shown in FIG. 9, having a diameter dimension a which is smaller than the diameter dimension A, for a longitudinal length dimension L. This means a sheath 25 is configured to include a sheath portion 25a having a larger diameter and a sheath portion 25b having a smaller diameter.

This allows the distal tip end of the sheath portion having a larger outer diameter dimension A to be placed at a desired position in the treatment instrument channel through the treatment instrument opening, for example in order to prevent any damage on the flexibility of the inserting section of the endoscope.

In FIG. 9, the treatment instrument is the biopsy forceps 2C. However, the treatment instrument is not limited to the biopsy forceps, and may be other treatment instruments which may have a sheath which includes a sheath portion 25a having a larger diameter and a sheath portion 25b having a smaller diameter.

In the second embodiment, the sheath 24 has the outer diameter dimension A. That is, the sheath 24 has a circular cross section with a diameter A. However, the sheath is not limited to have a circular cross section. In other words, for example as shown in FIG. 10A, and FIG. 10B, the sheath may have a generally elliptical front elevational shape and a generally elliptical cross section.

A high-frequency snare 3D shown in FIG. 10A has a sheath 26A which has a dimension X along its major axis and a dimension F along its minor axis. While, a high-frequency snare 3D1 shown in FIG. 10B has a sheath 26B which has a dimension Y along its major axis and a dimension F along its minor axis. This means the high-frequency snares 3D and 3D have the sheath 26A and the sheath 26B respectively both of which have the same dimension F along their minor axes.

The dimension F along their minor axes is set in consideration of a space G between the roller surfaces of the rollers 33a and 33b of the electric advancing and retracting device 30. That is, the sheaths 26A and 26B are placed so that the surfaces along their minor axes having the dimension T can be held under pressure in the space G defined by the roller surfaces of the rollers. This allows the outer surfaces along their minor axes of the sheaths 26A and 26B to be held under a predetermined pressure by the rollers which are separated by the space G. The sheaths 26A and 26B are made of a resin material having a flexibility, and include surfaces along their major axes having a fixed surface roughness.

In this way, in the treatment instrument which includes a sheath having an elliptical cross section, a dimension along its minor axis is set in consideration of the space between rollers of the electric advancing and retracting device, independently of a dimension along its major axis. This allows sheaths having different dimensions along their major axes to be held under pressure in the same way between the rollers of the electric advancing and retracting device.

It should be noted that in this embodiment, the dimension of the sheath along its minor axis is set in consideration of the space between the rollers. However, the dimension of the sheath along its major axis may be set in consideration of the space between the rollers. And, the sheath may have a flattened cross section which has opposing semi-circular portions and opposing linear portions, and the like. Also, a through-hole of the sheath may have a circular cross section, as well as an elliptical cross section or a flattened cross section depending on its outer profile. A through-hole having an elliptical cross section or a flattened cross section provides a wider cross section area than that having a circular cross section. In a configuration in which an operation wire is inserted in a through-hole, such a through-hole having a wider cross section area improves an operability of the operation wire, and in a configuration in which the through-hole serves as a lumen to supply solution, such a through-hole having a wider cross section area improves a property to supply solutions.

Having described the preferred embodiments of the invention referring to the accompanying drawings, it should be understood that the present invention is not limited to those precise embodiments and various changes and modifications thereof could be made by one skilled in the art without departing from the spirit or scope of the invention as defined in the appended claims.

## Claims

1. An endoscope treatment system in which a treatment instrument inserting section of a treatment instrument is moved by an advancing and retracting device, comprising:
a pair of rollers which are separated by a predetermined dimension in the advancing and retracting device; and
pressing force adjusting means for holding an outer surface of the treatment instrument inserting section under a predetermined pressure when the treatment instrument inserting section is held between the rollers.

2. The endoscope treatment system according to claim 1, wherein
the pressing force adjusting means is an adapter which is attached to the treatment instrument inserting section, and
the adapter comprises:
a through-hole through which the treatment instrument inserting section is inserted; and
a surface to be pressed which is held under pressure between the pair of rollers and pressed under a predetermined pressure by each of the rollers while being held under pressure.

3. The endoscope treatment system according to claim 2, wherein
the adapter is made of a material having a same flexibility, and the surface to be pressed thereof has a fixed surface roughness.

4. The endoscope treatment system according to claim 2, further comprising:
a removable section for combining the adapter and the treatment instrument inserting section as a unit.

5. The endoscope treatment system according to claim 4, wherein
the removable section comprises:
a circumferential groove formed in an inner surface of a through-hole at an end of the adapter; and
a circumferential projection formed on the treatment instrument to be engagedly fitted in the circumferential groove.

6. The endoscope treatment system according to claim 1, wherein
the pressing force adjusting means is a treatment instrument inserting section which has a circular cross section, and includes a circumferential surface as a surface to be pressed to be held under pressure between a pair of rollers independently of types of the treatment instrument.

7. The endoscope treatment system according to claim 1, wherein
the pressing force adjusting means is a treatment instrument inserting section which has an elliptical cross section, and includes either one of a surface along a minor axis of the ellipse or a surface along a major axis of the ellipse as a surface to be pressed to be held under pressure between a pair of rollers independently of types of the treatment instrument.

8. The endoscope treatment system according to claim 6 or 7, wherein
the treatment instrument inserting section is made of a material having a same flexibility, and the surface to be pressed thereof has a fixed surface roughness.

9. An endoscope treatment system, comprising:
a plurality of types of treatment instruments which include treatment instrument inserting sections to be inserted into a treatment instrument channel of an endoscope, the treatment instrument inserting sections having different outer diameter dimensions;
a plurality of types of adapters comprising through-holes through which the treatment instrument inserting sections having different outer diameter dimensions are inserted respectively, the adapters including surfaces to be pressed which have a fixed dimension when the adapters are attached to the treatment instrument inserting sections of the treatment instruments; and
an advancing and retracting device which includes a pair of rollers separated by a predetermined distance, and holds the surface to be pressed of the adapter attached to the treatment instrument inserting section under a predetermined pressure between the rollers, so that, with the adapter being held, a rotation of the rollers causes the adapter to advance and retract.

10. The endoscope treatment system according to claim 9, wherein
the adapters are made of a material having a same flexibility, and the surfaces to be pressed have a fixed surface roughness.

11. The endoscope treatment system according to claim 9, further comprising:
a removable section for combining the adapter and the treatment instrument inserting section as a unit, wherein
the removable section, comprises:
a circumferential groove formed in an inner surface of the through-hole at an end of the adapter; and
a circumferential projection formed on the treatment instrument to be engagedly fitted in the circumferential groove.

12. An endoscope treatment system, comprising:
a plurality of types of treatment instruments which include treatment instrument inserting sections to be inserted into a treatment instrument channel of an endoscope, the treatment instrument inserting sections having surfaces to be pressed which have a fixed dimension; and
an advancing and retracting device which includes a pair of rollers separated by a predetermined distance, and holds a surface to be pressed of the treatment instrument inserting sections under a predetermined pressure between the rollers, so that, with the surface to be pressed of the treatment instrument inserting section being held, a rotation of the rollers causes the treatment instrument inserting section to advance and retract.

13. A treatment instrument, comprising:
a treatment instrument inserting section to be inserted into a treatment instrument channel of an endoscope by an advancing and retracting device,
wherein the treatment instrument inserting section has a surface to be pressed which is held under pressure by a pair of rollers which are separated by a predetermined dimension in the advancing and retracting device.

14. An adapter to be attached to a treatment instrument inserting section of a treatment instrument, comprising:
a surface to be pressed which is held between a pair of rollers, the rollers being provided in an advancing and retracting device and being separated by a predetermined dimension; and
a through-hole having a diameter dimension corresponding to a diameter dimension of the treatment instrument inserting section, which is a dimension with remaining play between the pair of rollers.
